# EUROPEAN PATENT SPECIFICATION

(11) **EP 2 563 241 B1**
(45) Date of publication and mention of the grant of the patent: **25.12.2013**
(21) Application number: 11727522.2
(22) Date of filing: 27.04.2011
(51) Int. Cl.: A61B 17/11, A61B 17/115, A61F 2/06

(54) **Applicator for applying staples for end -to -end vascular grafts**
Applikator zur Applikation von Klammern für End-zu-End-Gefässersatz
Applicateur pour la pose d'agrafes pour des greffes vasculaires bout à bout

(30) Priority: 30.04.2010 IT BO20100271
(43) Date of publication of application: 06.03.2013
(73) Proprietor: NEWMAN MEDICAL KFT., 1146 Budapest (HU)
(72) Inventor: BORGHI, Enzo, I-40054 Budrio (IT)
(74) Representative: Conti, Marco
(86) International application number: PCT/IB2011/051832
(87) International publication number: WO 2011/135523

(56) References cited:
- WO-A1-2006/043129
- WO-A2-01/91628
- WO-A2-2005/063130
- DE-B3-102008 048 293

## Description

### Technical Field

This invention relates to an applicator for staples for end-to-end vascular grafts, in particular for grafts used to join end zones of body ducts.

In other words, the invention relates to an applicator for joining the staples making up a vascular graft during anastomosis operations in which two hollow structures consisting, for example, of blood vessels or, more generally, ducts for human body fluids, are surgically connected.

In a medical context, this operation regards two end portions of a duct for body fluids (for example, a blood vessel, a lymphatic vessel or other type of duct), and serves to connect the portions frontally or "end-to-end" in order to restore flow to the blood vessel or duct.

### Background Art

In effect, in surgical practice, the surgeon might for example have to cut a body duct in order to perform a particular operation; at a later stage, the end portions (or free ends) of the cut duct must be connected in order to restore the continuity of the duct. this is done by applying the graft staples to the two ends of the duct and joining them with a suitable connector.

To install these grafts, the surgeon first uses an applicator - the main object of this invention - which operates between the end of the duct and the staple to join the staple to the respective end edge of the duct quickly and precisely, and then an actuator to join the two end staples to the final connector.

The applicators available on the market today come in different kinds, depending also on the type of staples used.

A first solution is known from document WO2001/72232 (in the name of the same Applicant as this invention) where the annular staple is preassembled on the applicator.

The applicator is furnished with a movement unit for moving a probe which in turn guides into the duct a series of flexible hooked rods which, once opened in "umbrella" fashion, come into contact with the inside of the duct and hold it in place to allow the staple to slide axially over the outside of the duct. The staple is in turn associated with the duct by causing the hooks to penetrate into the walls of the duct by an axial movement opposite to the preceding superposing movement.

A second prior art solution is described in patent document WO2005/63130 (in the name of the same Applicant as this invention) where the applicator is used for a staple comprising a first ring furnished with deformable piercing legs and a second ring furnished with guide indentations by which the legs are directed towards the walls of the duct.

The applicator is positioned coaxially with the duct and basically comprises a nose-piece for precisely engaging the inside wall of the end portion of the duct and means for tightening the two rings to each other in a longitudinal direction so that the legs enter the walls of the duct.

In this case, the staple is placed over the end of the duct before inserting the nose-piece completely. The end of the duct with the staple on it is placed inside a hollow retaining body in which the nose-piece slides and in which there are means for tightening the two rings to each other by a movement parallel with the hollow body.

A third prior art solution is known from document WO2006/43129 (in the name of the same Applicant as this invention) where the applicator acts on staples composed of a first ring furnished with deformable piercing legs and a second ring furnished with holes through which the legs are made to pass in such a way as to guide them towards the walls of the duct. The two-part form of independent claim 1 is based on this document.

The applicator, in this case, is composed of a cylindrical body in which slides a cylindrical stem with a nose-piece designed to engage the inside surface of the end portion of the duct. The end portion of the cylinder has a first inside surface for abutting the free front edge of the end portion of the vessel (that is, of the duct) and a second hollow cylindrical element which accommodates both a part of the duct and the staple. Longitudinal movement of the second cylindrical element, combined with the presence of a further, second shaped abutment surface, makes it possible to move the two rings closer together and, at the same time, to guide the legs towards the duct walls, so as to attach the staple to the duct itself.

The applicators described above are not free of disadvantages, however.

The structure of these applicators is designed to operate along the same axis as the blood vessel (or duct), thereby creating two orders of problems.

First of all, the applicator must be positioned in a space between the two sections of the duct to be joined, which in practice gives the surgeon an extremely confined space to work in. That means a relatively large portion of the blood vessel must be extracted, creating serious discomfort for the patient.

Further, the surgeon has to move around the patient in order to be able to apply the other staple to the part of the duct still to be stapled, opposite the part stapled previously, with obvious inconvenience for the surgeon.

Yet another problem (at least for the applicators according to the second and third solutions described above) is due to the need for at least two surgeons to perform the steps of: fitting the nose-piece into the duct and then placing the duct, with the graft pre-fitted to it, into the (closed) end of the applicator.

This problem is due to the structure of the applicator which requires: a first surgeon to hold the applicator, which is open at one end, in position substantially coaxial with the end of the duct, while a second surgeon must manually insert the end of the duct itself into the end of the applicator and over the nose-piece; after doing that, the second surgeon must close the end of the applicator with the second fastening member to draw the rings together.

To this must be added the fact that the act of attaching the graft, that is, the pushing action by which the two rings are squeezed together, is performed practically "in the dark" (that is to say, without giving the surgeon the chance to see what he is doing): that is because the staple and the end of the duct are inside the (closed) cylindrical bodies which are movable relative to the applicator.

### Disclosure of the Invention

The aim of this invention is to overcome the above mentioned disadvantages by providing an applicator for staples for end-to-end vascular grafts which has a compact structure and a reduced working width.

Another aim of the invention is to provide an applicator for graft staples which is quick and easy to use on any end zone of the duct the staple is to be applied to.

A further aim of the invention is to provide a staple applicator that can be operated by a single operator working on both the staples to be applied without having to move around the patient.

A further aim of the invention is to provide a kit of elements which can be used to provide the applicator quickly and precisely with the working parts most suitable for the type of duct to be grafted.

These aims are fully achieved by the staple applicator for attaching the staples to the applicator according to the invention, as characterized in the appended claims.

More specifically, the applicator according to the invention comprises a nose-piece which can be inserted into an end zone of the body duct and positioned along a longitudinal axis, and two fastening members which can be drawn close together in order to abut a first annular body, provided with legs, and a second annular body in such a way that the legs are inserted into the duct wall.

According to the invention, the applicator comprises an arm having one end shaped in such a way as to support the nose-piece positioned perpendicularly to a transversal direction or axis of extension of the arm.

In other words, when the nose-piece is inserted into the end portion of the duct, the arm is positioned transversally to the position of the end zone of the duct itself. That makes it possible to reduce considerably the working space necessary for the operation of attaching the staple, allowing the surgeon not only to remain at a certain distance from the duct but also to minimize the portion of the duct that has to be extracted.

Also according to the invention, the second fastening member is movable along the transversal axis between a retracted position of non-interference with the end zone of the duct, and an extracted position where it is aligned with the nose-piece.

According to another aspect of the invention, the second fastening member is associated with the arm and movable along the same.

More specifically, the second fastening member comprises a forceps which is movable between an open position and closed position where the forceps surrounds (preferably completely) a portion of the end zone of the duct. In the extracted position, the forceps is positioned on the opposite side of the first fastening member with respect to the nose-piece, thereby defining two facing abutment zones for the two annular bodies, leaving the annular bodies in view.

This advantageously allows a single surgeon to perform all the operations needed to prepare the fastening members at a certain distance from the end of the duct and to be able not only to check visually that the fastening members have been prepared correctly but also to see the staple when it is attached.

According to another aspect of the invention, the first fastening member, which consists of a flat annular surface joined to the nose-piece, moves along a longitudinal direction (perpendicular to the transversal arm and coinciding with the axis of extension of the duct) between a rest position, with the nose-piece inserted into the end zone of the duct, and a working position where the nose-piece is advanced further inside the end zone of the duct and the first fastening member causes the two annular bodies to draw closer together thanks to contact with the closed forceps on the other side.

According to another aspect of the invention, the first fastening member, together with the nose-piece, forms an interchangeable cap on a supporting plate located on the end of the arm.

This advantageously makes it possible, prior to installation of the staple, to fit the arm rapidly and safely with the nose-piece and related fastening member of the size most suitable for the blood vessel.

In other words, the applicator according to the invention has a supporting arm for both the fastening members and for the nose-piece and which is positioned transversally to the duct. A single surgeon can position the nose-piece and activate the fastening members while remaining at a certain distance from the end of the duct and visually checking operations without the help of an assistant.

To obtain these results, the surgeon has a preparatory kit comprising: the applicator according to the invention; a set of nose-pieces with first fastening member to be placed on the supporting arm, each nose-piece being different in size; one or more testers provided with a magnet and used to manually test the different nose-pieces on the end zone of the duct to check the inside diameter of the duct; and lastly, a gauge to measure the outside diameter of the duct.

The kit thus allows rapid selection of the most suitable nose-piece to be used as well as the staples to be placed on the end zones of the duct.

### Brief Description of the Drawings

The technical features of the invention according to the above mentioned aims are clearly described in the claims below and its advantages are apparent from the detailed description which follows, with reference to the accompanying drawings, which illustrate a preferred non-limiting example embodiment of the invention and in which:
- Figure 1 is an exploded perspective view of an applicator according to the invention for applying staples to end-to-end grafts;
- Figures 2, 3 and 4 are plan views from above illustrating the applicator of Figure 1 with an operating arm in three different operating configurations;
- Figures 5 and 6 are side views showing the operating arm of the preceding figures where two fastening members supported by the arm are in two different working configurations;
- Figure 7 is a plan view from above, with some parts in cross section, illustrating a first operating step of installing a graft staple;
- Figure 8 is a cross section of the applicator in a second step of installing the graft staple;
- Figure 9 is an enlarged view, partly in cross section, showing a detail of the arm of the applicator of Figure 8;
- Figures 10 and 11 are cross sections illustrating, respectively, two duct ends provided with staples which can be installed with the applicator according to the invention and the duct ends after they have been completely joined by the graft;
- Figure 12 is a cross section of a detail referring to Figure 2;
- Figure 13 is a schematic front view showing a complete kit comprising an applicator according to the invention and a set of components for preparing and applying staples to the end zones of the duct.

### Detailed Description of the Preferred Embodiments of the Invention

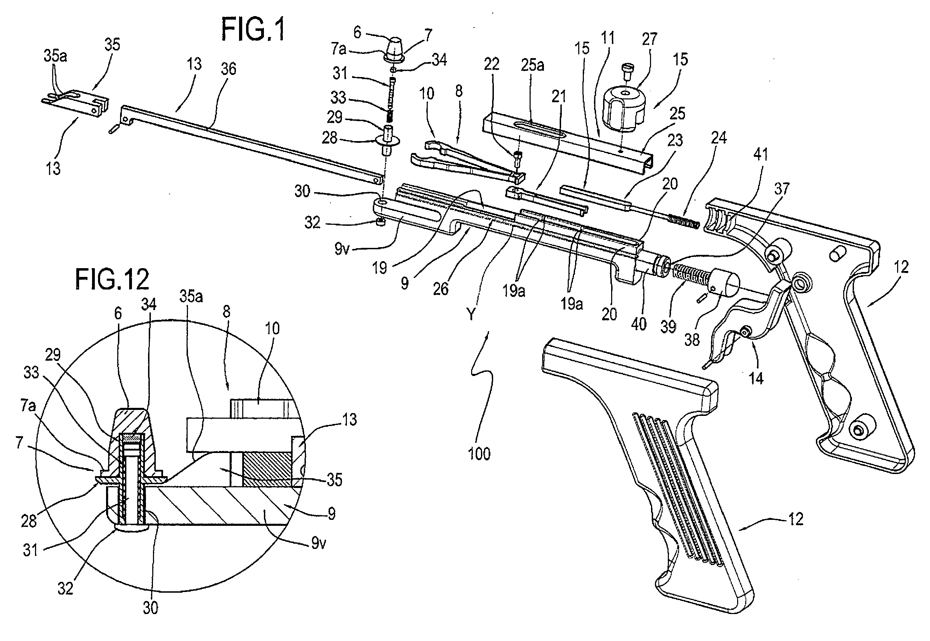

With reference to the accompanying drawings, in particular Figure 1, the applicator according to the invention, denoted in its entirety by the numeral 100, is used to attach staples 1 for end-to-end grafts 17 to end zones 2, 16 of one body duct or, if necessary, of another duct (such as a vein, an artery or a stretch of intestine, in particular of the human body).

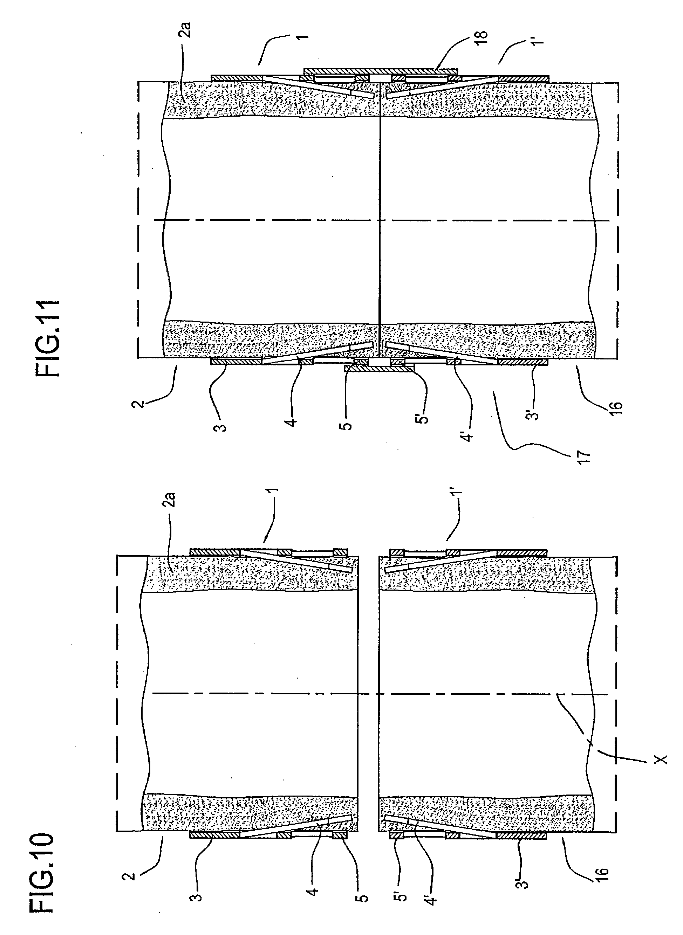

In this description, the term "graft" 17 means a set of elements comprising two staples 1, 1' which, once attached to the two end zones of the severed duct, allow the duct to be grafted by applying a connector 18 (illustrated schematically in Figure 11).

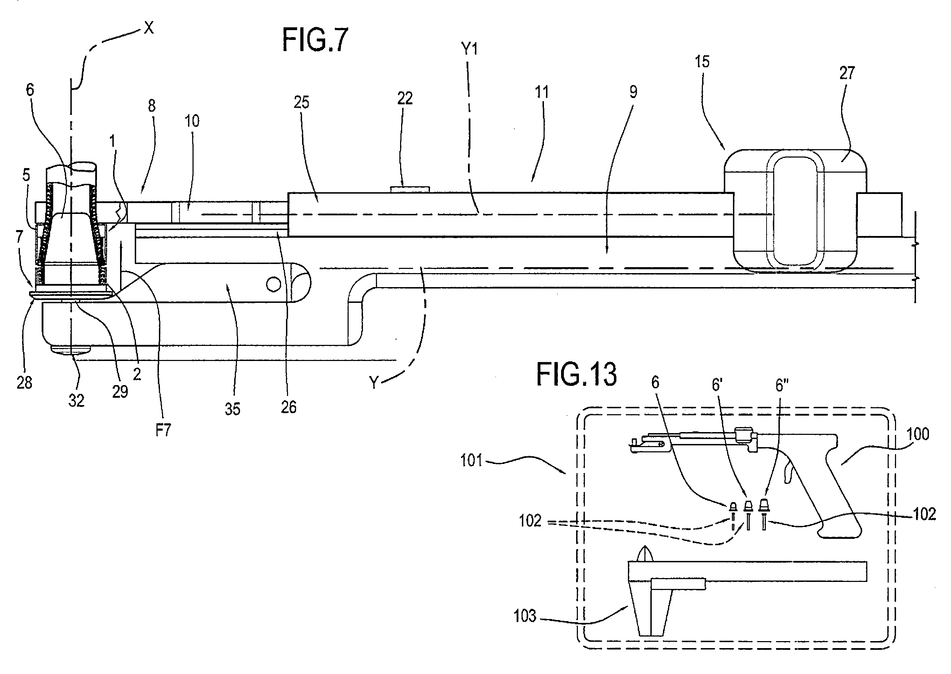

As shown in Figures 7 to 11, each staple 1, 1' has a first annular body 3 equipped with piercing legs 4 and a second annular body 5 for guiding the legs 4.

Obviously, the type of staples 1, 1' of the grafts 17 may differ in size and in the auxiliary elements for the three above-mentioned elements, without thereby limiting the scope of the invention.

Hereinafter, for simplicity, only one installable staple 1 will be described since the structure of the second staple 1' is the same as that of the first staple 1.

According to the invention, the applicator 100 comprises a nose-piece 6 configured to be inserted into the end zone 2 of the body duct and positioned along a longitudinal axis X, a first fastening member 7 associated with the nose-piece 6 and a second fastening member 8.

The first and second fastening members 7 and 8 can be drawn together to abut the first and the second annular body 3 and 5 in order to insert the legs 4 into the duct wall 2a (see also Figures 7 to 11).

Also according to the invention, the applicator 100 comprises an arm 9 having one end shaped in such a way as to support the nose-piece 6 positioned perpendicularly to a transversal direction or axis Y of extension of the arm 9.

This configuration allows the nose-piece 6 to be positioned in the end zone 3 while keeping the arm 9 perpendicular to the duct to be grafted.

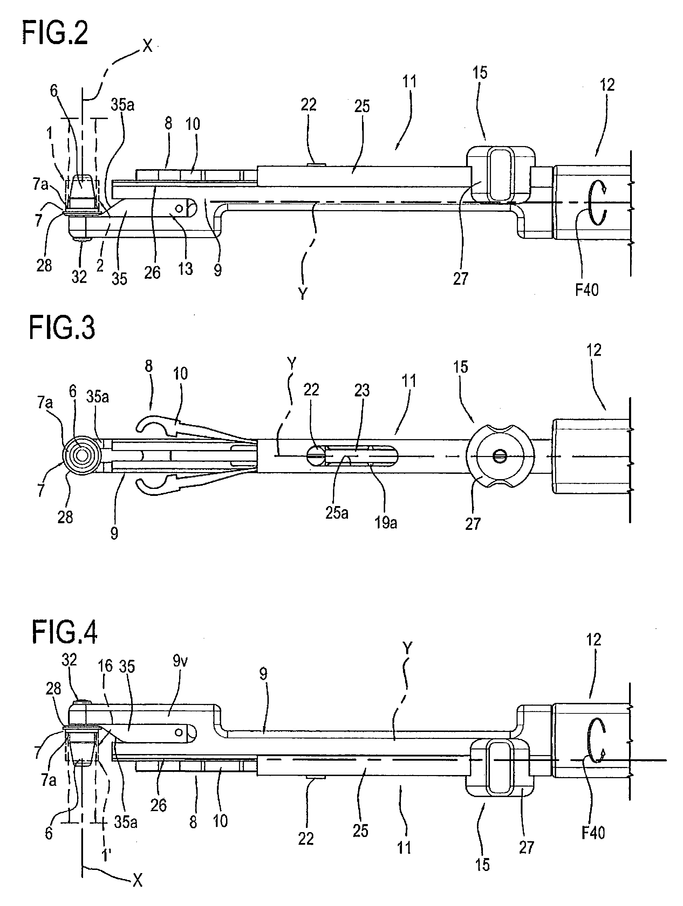

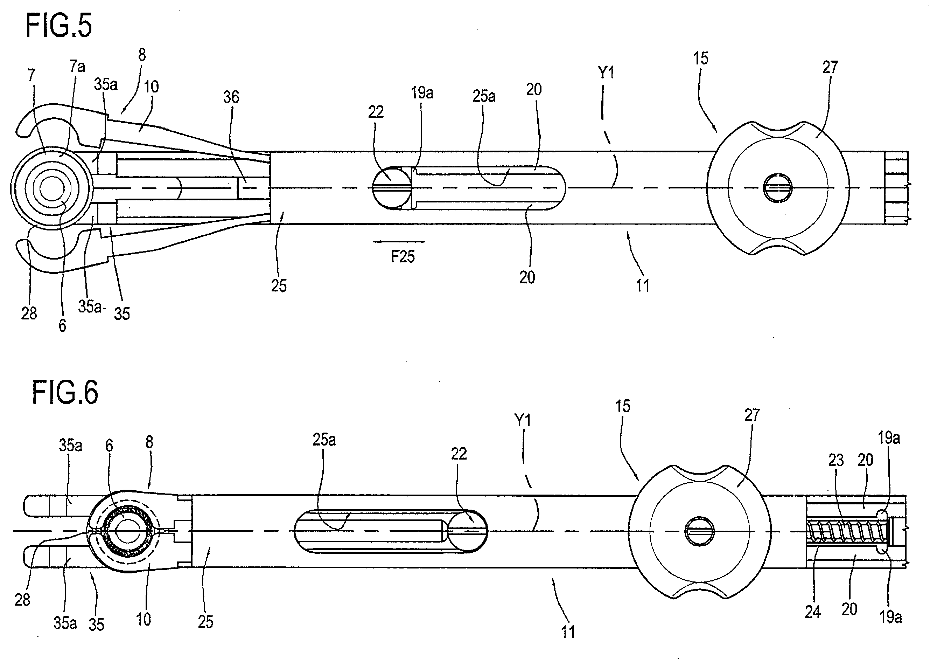

Again according to the invention, the second fastening member 8 is movable along the transversal direction (in this case denoted by an axis Y1) between a retracted position of non-interference with the end zone 2 of the duct (see Figures 2 to 4) and an extracted position where it is aligned with the nose-piece 6 (see Figures 5 to 9)

Preferably, as will become clearer as this description continues, the second fastening member 8 is movably coupled to the arm 9.

More specifically, the second fastening member 8 comprises a forceps 10 movable between an open position, where the end zone 2 of the duct can be inserted into the forceps 10, and a closed position, where the forceps 10 operatively surrounds a portion of the end zone 2 of the duct (see Figures 6 to 9).

Preferably, the forceps 10 in the closed position completely encircles the end zone 2.

As clearly shown in Figure 7, the forceps 10, in the extracted position, is located on the side opposite the first fastening member 7 with respect to the nose-piece 6.

The applicator 10 also comprises means 11 for kinematically coupling the opening and closing movement of the forceps 10 to the sliding movement of the same between the retracted and the extracted positions along the transversal axis Y1.

The axis Y1 is thus parallel to the direction or axis Y of extension of the arm 9.

According to another aspect of the invention, the kinematic means 11 form part of sliding means 15 by which the forceps 10 is made to slide along the arm 9 in both directions.

In effect, on a surface of the arm 9 there is formed a slot 19 which is partly delimited by protruding walls 20 forming a guide channel for the sliding means 15.

Looking in more detail, the sliding means 15 comprise a fastening member or supporting guide fork 21 associated at one end with the closed rear end of the forceps 10 through a coupling screw 22.

The fork 21 serves to keep the forceps 10 at a height above the slot 19 and, more specifically, in abutment against the end stretch of the arm 9, while the fork 21 is inside the slot 19 with its two arms facing towards the opposite inside walls of the selfsame slot 19.

On these inside walls of the slot 19 there are formed vertical indentations 19a which can be engaged by the arms of the fork 21 and which, as described below, indicate when the forceps 10 has reached a predetermined position during its backward and forward movement along the arm 10.

The slot 19 also accommodates a first slider 23 which is superposed over the fork 21 and interposed between the rear end of the forceps 10 and the closed end of the slot 19 on the side opposite, or distal from, the arm 9.

The rear portion of the first slider 23 is in the form of a rod with an auxiliary spring 24 fitted over it to apply a pushing action when the first slider 23 moves forward.

Obviously, the spring 24 is held back also thanks to the position of the fork 21 arms engaged in the indentations 19a.

The slot 19 is covered by a sliding element or second slider 25 which defines the above mentioned kinematic means 11.

The second slider 25 is substantially in the shape of an upturned U and is slidably associated with the free arms of the U on longitudinal guides 26 formed on the supporting arm 9.

The top of the second slider 25 is associated with an operating knob 27 by which the second slider 25 can be pushed or pulled.

The pushing or pulling action applied by means of the knob 27 moves the first slider 23 and, above all, the fork 21 thanks to a specially shaped slot 25a which is formed on the second slider 25 and which accommodates the head of the screw 22 that joins the forceps 10 and the fork 21, thereby causing the parts to slide along the arm 9.

In other words, the second slider 25 slides along the arm 9 in such a way as to:
- move from a withdrawn position to a first advanced position to shift the forceps 10 (by pulling) from the retracted to the extracted position, and
- from a first advanced position to a second advanced position, further forward than the first, in order to interact with a matchingly shaped surface of the forceps 10 causing the latter to move from the open to the closed position.

When the forceps 10 is in the extracted position, during the movement of the second slider 25 from the first to the second advanced position, the fork 21 prevents the forceps 10 from moving further in the direction of extraction along the axis Y1 thanks to abutment against the end wall of the slot 19.

In other words, therefore, to move the forceps 10 to the extracted position, the surgeon acts on the knob 27 to make the second slider 25 advance along the arm (see arrow F25, Figures 6, 7 and 8). This movement causes abutment against the head of the screw 22 and determines the pushing action on the fork 21 and on the forceps 10, forcing the latter to advance along the arm 9.

The movement of the forceps 10 and of the fork 21 (associated with the forceps 10) is assisted by the first slider 23 thanks to the spring 24 which, being compressed, applies a force that pushes the first slider 23 forward.

As it moves forward, the fork 21 meets the front wall of the slot 19 and, in addition, snaps into the front indentations 19a formed in the slot 19 itself, thereby indicating, in practice, that the group of elements in the slot 19 has moved as far forward as possible and the forceps 10 is aligned with the nose-piece 6 but in the open position (see Figure 5).

At this point, pushing the second slider 25 further forward causes abutment against the matchingly shaped surfaces of the forceps 10 (in practice, the outside surfaces of the arms) and makes the arms of the forceps 10 close elastically around the end zone 2 of the duct (see Figure 6).

To release the forceps 10 from the duct, the operations described above are performed in reverse order, that is to say:
- first withdrawal of the second slider 25, causing the arms of the forceps 10 to open;
- second withdrawal of the second slider 25, causing withdrawal also of the forceps 10, of the fork 21 and of the first slider 23.

Also according to the invention, the first fastening member 7 is movable relative to the second fastening member 8 perpendicularly to the transversal axis Y of extension of the arm 9, along a longitudinal direction (see arrow F7, Figures 7, 8 and 9), coinciding with the axis X of duct extension.

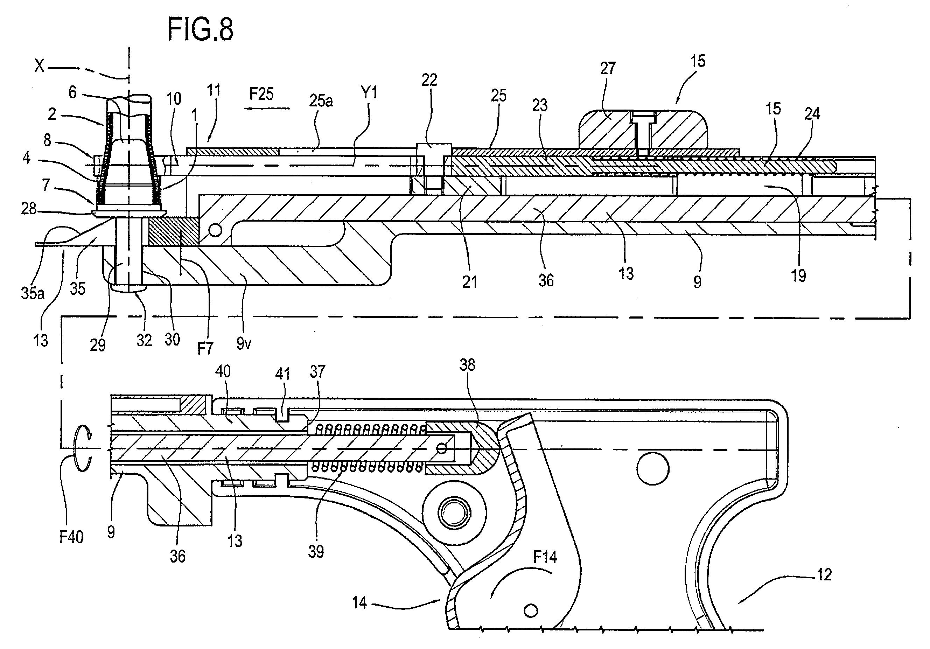

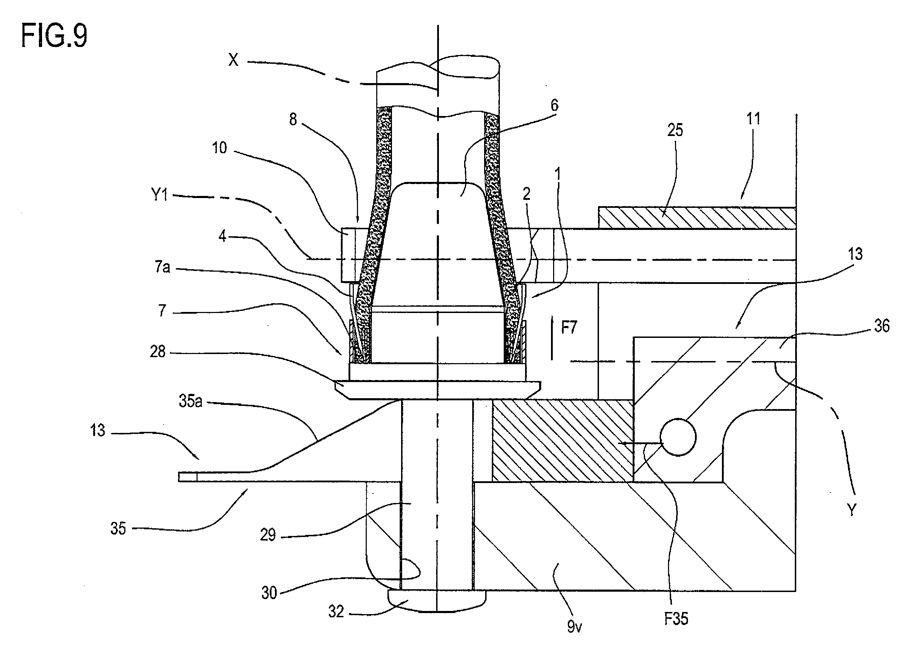

This movement of the first fastening member 7 is from a rest position where the nose-piece 6 is coupled in the end zone 2 of the duct (see Figure 7) and a working position, where the nose-piece 6 is further inside the end zone 2 of the duct and the first fastening member 7 causes the two annular bodies 3 and 5 to be drawn together (see Figures 8 and 9).

In light of this, the applicator 100 comprises:
- a supporting plate 28 associated with the end of the supporting arm 9;
- an annular member 7a associated with a base of the nose-piece 6 and forming the first fastening member 7.

In practice, the nose-piece 6 and the annular member 7a constitute a cap 6 which is associated removably with the supporting plate 28 so it can be interchanged with other caps.

More specifically, the plate 28 is integral at the centre of it to a cylinder 29 whose bottom can be coupled in a through hole 30 made in the hook-shaped front end 9u of the arm 9.

This end of the arm 9 allows the cap 6, that is, the annular member 7a, to lie in plane different from the forceps 10.

The cylinder 29 is fastened to the end of the arm 9 by a screw 31, which passes through the inside of the cylinder 29 itself, and a lock nut 32 located on the opposite surface of the end of the arm 9.

In addition to that, the screw 31 has a second spring 33 fitted round it and stopped at the ends of it by the screw 31 itself and by the nut 32, in such a way as to keep the plate 28 in a lowered position on the arm 9 in a non-operating position.

As may be observed in the detail shown in Figure 12, the nose-piece 6 is mounted on the plate 28, encircling the top of the cylinder 29, and held in place by a magnet 34 located internally between the nose-piece 6 and the head of the screw 31 and acting between the two to keep the nose-piece 6 securely in position.

According to another aspect of the invention, the first fastening member 7, that is, the annular member 7a of the nose-piece 6, is driven by cam movement means 13 located along the arm 9, parallel to the kinematic means 11 for movement of the forceps 10, able to be operated manually using a control lever 14 located on an operating handgrip 12 to which the arm 9 is mounted (see Figures 1 and 8).

In light of this, the cam means 13 comprise a slide 35 located on the proximal end 9u of the arm 9 and a control rod 36 of the slide 35.

The control rod 36 is seated in a housing 37 passing longitudinally through the arm 9 and with its distal end extends into the handgrip 12.

Here, the rod 36 is provided with an end cap 38 positioned in face contact with the portion of the control lever 14 which is inside the handgrip 12.

Fitted over the part of the rod 36 that protrudes from the arm 9 and extends into the handgrip 12 there is a third spring 39 held between the rear end of the arm 9 and the cap 38, the third spring 39 serves to keep the rod 36 in a position which opposes the inside of the lever 14.

The front end of the rod 36, protruding from the housing 37 of the arm 9 (in the vicinity of the hooked proximal end 9u of the arm 9 itself) is connected to the slide 35 that runs along the end 9u of the arm 9.

The slide 35 has an inclined front surface 35a whose straight end stretch is located under the above mentioned plate 28 (see Figure 7). In addition to that, the front surface 35a has a forked structure so it can be positioned under the plate 28 and, at the same time, does not interfere with the cylinder 29.

In practice, when the surgeon acts on the part of the lever 14 forming the trigger, so to speak, the lever 14 itself is made to rotate (see arrow F14) thereby pushing the control rod 36 which in turn causes the slide 35 to advance (see arrow F35).

The advancing movement of the slide 35 along the transversal axis Y causes the plate 28 to move away from the end 9u of the arm 9 and thus also of the nose-piece 6 and of the respective first fastening member 7: this is accomplished to the special shape of the front surface 35a of the slide 35.

The movement continues until the plate 28 is substantially on the straight top surface of the slide 35 (see Figures 8 and 9).

The movement of the plate 28 bearing the nose-piece 6, when the forceps 10 is also aligned with the nose-piece 6 and closed around the duct, causes the staple 1 to be attached to the end zone 2 of the duct (as shown in Figure 10).

To be able to repeat the same operation on the other end zone 16 of the duct, the supporting arm 9 is movably connected, at the end opposite the one that supports the nose-piece 6, to the operating handgrip 12 to rotate about its axis Y of extension and to place the two fastening members 7 and 8 and the nose-piece 6 in the right position for installing the second staple 1'.

More specifically, the rear end of the arm 9 has a cylindrical protrusion 40 which is provided with a circular groove and which can be engaged with a circular seat 41 on the handgrip 12. The circular groove engages an axial retaining ring 41 to hold the arm 9 against the handgrip 12 but with the possibility of rotating about the transversal axis Y (see arrow F40).

This invention, as shown in Figure 13, provides an application kit 101 comprising preferably:
- the applicator 100 described up to here but without the nose-piece 6 and the respective annular member 7a;
- a set of nose-pieces 6, 6', 6" (the number varies according to the range of duct sizes to be covered);
- preferably one or more testers 102 each composed of a grip rod and provided with a magnetic end to be joined temporarily to a nose-piece 6 in order to check whether the nose-piece 6 selected is of the correct size for the inside diameter of the end zone 2 to be grafted; and
- a gauge 103 for measuring the outside diameter of the duct in the vicinity of its end zone 2 in order to choose the staple 1,1' to be installed.

Preferably, all the components in the kit are of the disposable type.

An exemplary method for applying the staple 1 for an end-to-end vascular graft 17 to the end zone 2 of a duct, is also disclosed.

The exemplary method comprises the steps of:
- choosing the staple 1 to be placed on the first end zone 2;
- choosing the nose-piece 6 and first fastening member 7 most suitable for installing the staple 1 according to the size of the duct to be grafted;
- setting the selected nose-piece 6 on the cylinder 29, which holds it in place thanks also to the magnet 34;
- positioning on the end zone 2 of the duct, the staple 1, that is, the first annular body 3 equipped with piercing legs 4 and the second annular body 5 for guiding the legs 4;
- inserting the nose-piece 6 into the first end zone 2 of the body duct and positioning it along the longitudinal axis X (see Figure 2) in such a way as to position the first fastening member 7 associated with the nose-piece 6 so it abuts one of the annular bodies 3, 5;
- shifting a second fastening member 8 (the forceps 10) along the transversal direction or axis Y from a retracted position of non-interference with the end zone 2 of the duct (Figure 2) to an extracted position where it is aligned with the nose-piece 6 for abutting the other annular body 5, 3 (Figures 5, 6 and 7);
- drawing the first and second fastening members 7, 8 together along the longitudinal axis X in order to abut the respective annular bodies 3, 5 and insert the legs 4 of the first annular body 3 into the duct wall 2a guided by the second annular body 5, thereby causing the staple 1 to be applied to the first end zone 2 of the duct (Figures 8, 9 and 10).

Preferably, the step of applying the first staple 1 comprises a movement of the first fastening member 7, that is, of the nose-piece 6 with the respective annular surface, towards the forceps 10 and along the longitudinal axis X.

As already mentioned, the step of positioning the forceps 10 on the first end zone 2 of the duct comprises moving the forceps 10 in the open position closer until aligning the forceps 10 with the nose-piece 6 to define the second fastening member 8, and then shifting the forceps 10 on the first end zone 2 to a closed position around the first end zone 2, leaving in view at least a part of the two annular bodies 3, 5.

At this point, that is to say, with the first staple 1 installed, the surgeon can release the lever 14 on the handgrip 12 to allow the nose-piece 6 and the first fastening member 7 to move back, under the action of the second spring 33, to the position where they are close to the end of the arm 9.

With the other hand, the surgeon re-opens the forceps 10 and using the knob 27, pulls it back to the withdrawn configuration.

The exemplary method may also comprises a step of rotating the arm 9 about the transversal axis X of extension of the arm itself before inserting the nose-piece 6, thus

At this point, the exemplary method comprises a step of positioning on the second end zone 16 of the duct, the second staple 1', consisting of a first annular body 3' equipped with piercing legs 4' and a second annular body 5' for guiding the legs 4'.

After positioning the second staple 1' the surgeon, as mentioned above, performs a step of rotating the supporting arm 9 through a suitable angle about the axis Y so as to prepare the fastening members 7 and 8 and the nose-piece 6 for the above mentioned steps of positioning and applying the second staple 1' on the second end zone 16 of the duct in a manner similar to that described above, thereby preparing the ends 2 and 16 for the graft, as shown in Figure 10.

As last step, the surgeon applies the connector 18 to the two staples 1 and 1' (see Figure 11) to join the duct through a respective actuator (not illustrated because it does not form part of the invention).

Preferably, the above mentioned exemplary step of choosing the staples 1, 1' to be placed on the duct end zones may be preceded by a step of measuring the outside diameter of the duct.

Preferably, this measuring step 1 may be performed using a suitable gauge 103.

Preferably, the step of choosing the nose-piece 6 to be fitted to the applicator 100 may comprise a step of measuring the inside diameter of the end zone.

Preferably, this measuring step may be performed by inserting the selected nose-piece 6 mounted on the above mentioned tester 102 into the end zone concerned.

Preferably, this step may be performed manually by the surgeon to check that the nose-piece 6 goes in and out of the end zone freely if the selected nose-piece 6 is the correct one, the nose-piece 6 itself must go in and out without coming away from the tester 102.

The applicator for grafting vascular ducts as described above thus fully achieve the above mentioned aims.

The structure of the applicator of this application has a multiplicity of advantages, including:
- reducing the space necessary for installation, thanks to the "transversal" geometry of the arm, allowing at the surgeon to perform all the steps in the installation process at a certain distance from the patient;
- practicality of use because the means for moving the forceps and the nose-piece with the annular member are conveniently located along the supporting arm or on the handgrip, allowing installation to be performed easily by a single surgeon;
- use of a nose-piece and forceps well spaced from each other, allowing the surgeon a clear view for visually checking the installation operation in all its stages.

It will be understood that the invention described is susceptible of industrial application and may be modified and adapted in several ways without thereby departing from the scope of the inventive concept.

## Claims

1. An applicator for applying a staple (1) for end-to-end vascular grafts to an end zone (2) of a body duct, the staple (1) having a first annular body (3) equipped with piercing legs (4) and a second annular body (5) for guiding the legs (4), comprising:
- a nose-piece (6) configured to be inserted into the end zone (2) of the body duct and positioned along a longitudinal axis (X);
- a first fastening member (7), associated with the nose-piece (6), and a second fastening member (8) which can be drawn together to abut the first annular body (3) and the second annular body (5), in such a way that the legs (4) of the first annular body (3) are inserted into the duct wall (2a) and are guided by the second annular body (5),
**characterized in that** it comprises an arm (9) having one end shaped in such a way as to support the nose-piece (6) positioned perpendicularly to a transversal direction (Y) of extension of the arm (9), the second fastening member (8) being movable along the transversal direction between a retracted position of non-interference with the end zone (2) of the duct, and an extracted position where it is aligned with the nose-piece (6).

2. The applicator according to claim 1, wherein the second fastening member (8) is movably coupled to the arm (9).

3. The applicator according to claim 1 or 2, wherein the second fastening member (8), in the extracted position, is located on the side opposite the first fastening member (7) with respect to the nose-piece (6).

4. The applicator according to any of the foregoing claims, wherein the second fastening member (8) comprises forceps (10) movable between an open position where the body duct can be inserted into the forceps (10), and a closed position where the forceps (10) operatively encircle the end zone (2) of the duct completely.

5. The applicator according to claim 4, comprising means (11) for kinematically coupling an opening and closing movement of the forceps (10) with a sliding movement of the same between the retracted and the extracted position.

6. The applicator according to claim 5, wherein the kinematic means (11) comprise:
- a slidable element (25) coupled to the arm (9), in such a way that it can move from a withdrawn position to a first advanced position to shift the forceps (10) by pulling them from the retracted to the extracted position, and from the first advanced position to a second advanced position, further forward than the first, in order to interact with a matchingly shaped surface of the forceps (10) causing the latter to move from the open to the closed position;
- a fastening member (21) associated with the arm (9) to prevent movement of the forceps (10) in the extraction direction along the axis of extension of the arm (9), when the forceps (10) are in the extracted position during the movement of the slidable element (25) from the first advanced position to the second.

7. The applicator according to any of the foregoing claims, wherein the first fastening member (7) is movable relative to the second fastening member (8) perpendicularly to the transversal axis (Y) of extension of the arm (9), along the longitudinal axis (X) of extension of the body duct end zone (2) coupled to the nose-piece (6), thereby causing the two annular bodies (3, 5) to be drawn together.

8. The applicator according to claim 7, comprising cam-like means (13) which are configured to move the first fastening member (7), are coupled to the arm (9) and are able to be operated manually using a control lever (14) located on an operating handgrip (12) for supporting the aim (9).

9. The applicator according to any of the foregoing claims, comprising:
- a supporting plate (28) associated with the end of the supporting arm (9);
- an annular member (7a) associated with a base of the nose-piece (6) and forming the first fastening member (7), the nose-piece (6) and the annular member (7a) constituting a cap (6) associated removably with the supporting plate (28) so it can be interchanged with other caps.

10. The applicator according to any of the foregoing claims, comprising a handgrip (12) for operating the applicator (100), the supporting arm (9) being movably connected, at the end of it opposite the end that supports the nose-piece (6), to the handgrip (12) in order to rotate about the axis of extension (Y).

## Patentansprüche

1. Applikator zur Applikation einer Klammer (1) für End-zu-End-Gefäßersatz an einem Endbereich (2) eines Körperkanals, wobei die Klammer (1) einen ersten ringförmigen Körper (3), der mit Durchstechschenkeln (4) ausgestattet ist, und einen zweiten ringförmigen Körper (5) zum Führen der Schenkel (4) aufweist, umfassend:
- ein Kegelstück (6), das dazu konfiguriert ist, in den Endbereich (2) des Körperkanals eingeführt und entlang einer Längsachse (X) positioniert zu werden;
- ein erstes Befestigungsglied (7), das mit dem Kegelstück (6) verbunden ist, und ein zweites Befestigungsglied (8), die zusammengezogen werden können, um am ersten ringförmigen Körper (3) und am zweiten ringförmigen Körper (5) so anzustoßen, dass die Schenkel (4) des ersten ringförmigen Körpers (3) in der Kanalwand (2a) eingeführt sind und vom zweiten ringförmigen Körper (5) geführt werden;
**dadurch gekennzeichnet, dass** er einen Arm (9) umfasst, aufweisend ein Ende, das so geformt ist, dass es das Kegelstück (6), das lotrecht zu einer transversalen Richtung (Y) der Ausdehnung des Arms (9) positioniert ist, trägt, wobei das zweite Befestigungsglied (8) entlang der transversalen Richtung zwischen einer eingezogenen Position des Nicht-Eingreifens in den Endbereich (2) des Kanals und einer ausgezogenen Position, in der es mit dem Kegelstück (6) ausgerichtet ist, bewegbar ist.

2. Applikator nach Anspruch 1, wobei das zweite Befestigungsglied (8) bewegbar mit dem Arm (9) verkuppelt ist.

3. Applikator nach Anspruch 1 oder 2, wobei das zweite Befestigungsglied (8) in der ausgezogenen Position in Bezug auf das Kegelstück (6) an der Seite gegenüber dem ersten Befestigungsglied (7) liegt.

4. Applikator nach einem der vorangehenden Ansprüche, wobei das zweite Befestigungsglied (8) eine Klemme (10) umfasst, die zwischen einer offenen Position, in der der Körperkanal in die Klemme (10) eingeführt werden kann, und einer geschlossenen Position, in der die Klemme (10) wirksam den Endbereich (2) des Kanals vollständig umgibt, bewegbar ist.

5. Applikator nach Anspruch 4, umfassend Mittel (11) zum kinematischen Verkuppeln einer Öffnungs- und Schließbewegung der Klemme (10) mit einer Verschiebungsbewegung dieser zwischen der eingezogenen und ausgezogenen Position.

6. Applikator nach Anspruch 5, wobei die kinematischen Mittel (11) Folgendes umfassen:
- ein verschiebbares Element (25), das so mit dem Arm (9) verkuppelt ist, dass es sich von einer zurückgezogenen Position in eine erste vorgerückte Position bewegen kann, um die Klemme (10) durch Ziehen von der eingezogenen in die ausgezogene Position zu verlagern, und von der ersten vorgerückten Position in eine zweite vorgerückte Position, die weiter vorn als die erste ist, um mit einer passend geformten Oberfläche der Klemme (10) zu interagieren, wodurch ihre Bewegung von der offenen in die geschlossene Position bewirkt wird;
- ein Befestigungsglied (21), das mit dem Arm (9) verbunden ist, um die Bewegung der Klemme (10) in der Auszugsrichtung entlang der Ausdehnungsachse des Arms (9) zu verhindern, wenn die Klemme (10) während der Bewegung des verschiebbaren Elements (25) von der ersten vorgerückten Position in die zweite in der ausgezogenen Position ist.

7. Applikator nach einem der vorangehenden Ansprüche, wobei das erste Befestigungsglied (7) relativ zum zweiten Befestigungsglied (8) lotrecht zur transversalen Achse (Y) der Ausdehnung des Arms (9) entlang der Längsachse (X) der Ausdehnung des mit dem Kegelstück (6) verkuppelten Endbereichs (2) des Körperkanals bewegbar ist, wobei das Zusammenziehen der beiden ringförmigen Körper (3, 5) verursacht wird.

8. Applikator nach Anspruch 7, umfassend nockenähnliche Mittel (13), die zum Bewegen des ersten Befestigungsglieds (7) konfiguriert sind, mit dem Arm (9) verkuppelt sind und dazu fähig sind, mithilfe eines Steuerhebels (14) manuell bedient zu werden, der an einem Bedienhandgriff (12) zum Tragen des Arms (9) liegt.

9. Applikator nach einem der vorangehenden Ansprüche, umfassend:
- eine Trägerplatte (28), die mit dem Ende des Trägerarms (9) verbunden ist;
- ein ringförmiges Glied (7a), das mit einer Basis des Kegelstücks (6) verbunden ist und das erste Befestigungsglied (7) definiert, wobei das Kegelstück (6) und das ringförmige Glied (7a) eine Kappe (6) bilden, die mit der Trägerplatte (28) abnehmbar verbunden ist, sodass sie mit anderen Kappen ausgetauscht werden kann.

10. Applikator nach einem der vorangehenden Ansprüche, umfassend einen Handgriff (12) zum Bedienen des Applikators (100), wobei der Trägerarm (9) an seinem Ende gegenüber dem Ende, das das Kegelstück (6) trägt, bewegbar an den Handgriff (12) angeschlossen ist, um um die Ausdehnungsachse (Y) zu rotieren.

## Revendications

1. Applicateur pour la pose d'une agrafe (1) pour des greffes vasculaires bout à bout à une zone d'extrémité (2) d'un conduit corporel, l'agrafe (1) ayant un premier corps annulaire (3) doté de pieds de perçage (4) et un second corps annulaire (5) servant à guider les pieds (4), comprenant :
- une pièce d'extrémité (6) configurée pour être insérée dans la zone d'extrémité (2) du conduit corporel et positionnée le long d'un axe longitudinal (X) ;
- un premier élément de fixation (7), associé à la pièce d'extrémité (6), et un second élément de fixation (8) pouvant être amenés ensemble pour se mettre en butée avec le premier corps annulaire (3) et le second corps annulaire (5), de sorte que les pieds (4) du premier corps annulaire (3) soit insérés dans la paroi de conduit (2a) et guidés par le second corps annulaire (5),
**caractérisé en ce qu'**il comprend un bras (9) disposant d'une extrémité ayant une forme telle à soutenir la pièce d'extrémité (6) positionnée perpendiculairement à une direction transversale (Y) d'extension du bras (9), le second élément de fixation (8) étant mobile le long de la direction transversale entre une position rétractée de non interférence avec la zone d'extrémité (2) du conduit, et une position d'extraction dans laquelle il est aligné avec la pièce d'extrémité (6).

2. Applicateur selon la revendication 1, dans lequel le second élément de fixation (8) est accouplé de façon mobile au bras (9).

3. Applicateur selon les revendications 1 ou 2, dans lequel le second élément de fixation (8), dans la position d'extraction, est situé sur le côté opposé au premier élément de fixation (7) par rapport à la pièce d'extrémité (6).

4. Applicateur selon l'une quelconque des revendications précédentes, dans lequel le second élément de fixation (8) comprend une pince (10) mobile entre une position ouverte dans laquelle le conduit corporel peut être inséré dans la pince (10), et une position fermée dans laquelle la pince (10) entoure complètement de façon fonctionnelle la zone d'extrémité (2) du conduit.

5. Applicateur selon la revendication 4, comprenant des moyens (11) pour accoupler cinématiquement un déplacement d'ouverture et de fermeture de la pince (10) avec un déplacement coulissant de ladite pince entre la position rétractée et d'extraction.

6. Applicateur selon la revendication 5, dans lequel les moyens cinématiques (11) comprennent :
- un élément pouvant coulisser (25) accouplé au bras (9), de sorte qu'il puisse se déplacer d'une position de retrait à une première position avancée pour déplacer la pince (10) en la tirant de la position rétractée à la position d'extraction, et de la première position avancée à une seconde position avancée, plus en avant par rapport à la première, afin d'interagir avec une surface à la forme adaptée de la pince (10) obligeant cette dernière à se déplacer de la position ouverte à la position fermée ;
- un élément de fixation (21) associé au bras (9) pour empêcher le déplacement de la pince (10) dans la direction d'extraction le long de l'axe d'extension du bras (9) lorsque la pince (10) est dans la position d'extraction lors du déplacement de l'élément pouvant coulisser (25) de la première à la seconde position avancée.

7. Applicateur selon l'une quelconque des revendications précédentes, dans lequel le premier élément de fixation (7) est mobile par rapport au second élément de fixation (8) perpendiculairement à l'axe transversal (Y) d'extension du bras (9), le long de l'axe longitudinal (X) d'extension de la zone d'extrémité (2) du conduit corporel accouplée à la pièce d'extrémité (6), causant ainsi que les deux corps annulaires (3, 5) peuvent être amenés ensemble.

8. Applicateur selon la revendication 7, comprenant des moyens en forme de came (13) étant configurés pour déplacer le premier élément de fixation (7), accouplés au bras (9) et pouvant être actionnés manuellement en utilisant un levier de commande (14) situé sur une poignée de manoeuvre (12) pour soutenir le bras (9).

9. Applicateur selon l'une quelconque des revendications précédentes, comprenant :
- une plaque de support (28) associée à l'extrémité du bras de support (9) ;
- un élément annulaire (7a) associé à la base d'une pièce d'extrémité (6) et formant le premier élément de fixation (7), la pièce d'extrémité (6) et l'élément annulaire (7a) constituant un capuchon (6) associé de manière amovible à la plaque de support (28) de sorte qu'il puisse être interchangeable avec d'autres capuchons.

10. Applicateur selon l'une quelconque des revendications précédentes, comprenant une poignée (12) pour manoeuvrer l'applicateur (100), le bras de support (9) étant relié de façon mobile à l'extrémité opposée de celle supportant la pièce d'extrémité (6) par rapport à la poignée (12) afin de tourner autour de l'axe d'extension (Y).
